(19) Europäisches Patentamt
European Patent Office
Office européen des brevets

(11) Publication number: **0 000 355**
**B1**

(12) **EUROPEAN PATENT SPECIFICATION**

(45) Date of publication of patent specification: **06.01.82**

(21) Application number: **78100274.6**

(22) Date of filing: **29.06.78**

(51) Int. Cl.³: **C 07 D 209/14,**
C 07 D 401/12,
A 61 K 31/40

(54) **New indole derivatives, processes for their preparation, and pharmaceutical compositions containing them.**

(30) Priority: **12.07.77 CH 8589/77**
**20.03.78 CH 3008/78**

(43) Date of publication of application:
**24.01.79 Bulletin 79/2**

(45) Publication of the grant of the European patent:
**06.01.82 Bulletin 82/1**

(84) Designated Contracting States:
**BE CH DE FR GB LU NL SE**

(56) References cited:
**Chemical Abstract (1974) vol. 81 — 1051 81 k.**

(73) Proprietor: **SANDOZ AG**
**Lichtstrasse 35**
**CH-4002 Basel (CH)**

(72) Inventor: **Stadler, Paul, Dr.**
**Jakobsweg 7**
**CH-4105 Biel-Benken (CH)**
Inventor: **Troxler, Franz, Dr.**
**Drosselstrasse 39**
**CH-4103 Bottmingen (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 000 355 B1

# New indole derivatives, processes for their preparation, and pharmaceutical compositions containing them

The present invention relates to new indole derivatives, processes for their preparation, and pharmaceutical compositions containing them.

In accordance with the invention there are provided new compounds of formula I

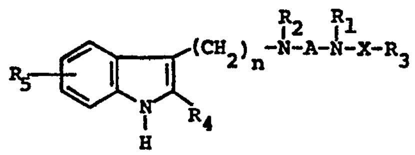

(I)

wherein n is 2 or 3,

either A is trimethylene optionally substituted by $(C_{1-4})$ alkyl or 1,4-cyclohexylidene and $R_1$ is hydrogen or $(C_{1-5})$ alkyl,

or A together with $R_1$ and the nitrogen atom to which $R_1$ is bound, form a 4-piperidyl radical,

$R_2$ is hydrogen or $(C_{1-5})$ alkyl,

$R_3$ is $(C_{1-4})$ alkyl; $(C_{3-8})$cycloalkyl; amino; $(C_{1-4})$alkylamino; di$(C_{1-4})$alkylamino; phenylamino wherein the phenyl ring is unsubstituted or mono-, di- or trisubstituted independently by halogen, $(C_{1-4})$alkyl, $(C_{1-4})$alkoxy or di$(C_{1-4})$alkylamino; phenyl or benzyl wherein the phenyl rings are unsubstituted or mono-, di- or trisubstituted independently by halogen, hydroxy, $(C_{1-4})$alkyl, $(C_{1-4})$alkoxy or di-$(C_{1-4})$alkylamino; 2-,3- or 4-pyridylmethyl; or an aromatic 5- or 6-membered heterocycle containing one heteroatom chosen from nitrogen, oxygen or sulphur and optionally an additional one or two nitrogen hetero atoms,

$R_4$ is hydrogen, chlorine, bromine or $(C_{1-4})$alkyl,

$R_5$ is hydrogen, $(C_{1-4})$alkyl, $(C_{1-4})$alkoxy or $(C_{1-4})$alkylthio,

and X is —CO— or —CS—.

Any alkyl, alkoxy or alkylthio radical in any of the above significances has preferably two carbon atoms, and signifies especially methyl, methoxy or methylthio. Halogen means fluorine, chlorine, bromine or iodine, especially chlorine.

When A is 1,4-cyclohexylidene, this may be cis or trans-1,4-cyclohexylidene.

When A is optionally substituted trimethylene, this is preferably either unsubstituted or mono-substituted, conveniently at the middle carbon atom.

When $R_1$ and $R_2$ are chosen from hydrogen or alkyl, these are preferably alkyl.

Conveniently A is optionally substituted trimethylene or 1,4-cyclohexylidene. Preferably A is optionally substituted trimethylene.

When $R_3$ is or contains a dialkylamino radical, the alkyl groups are preferably the same. When $R_3$ is an optionally substituted phenyl or phenylamino radical, the substituents are conveniently identical. Conveniently these radicals are unsubstituted or mono-substituted preferably in the para position. When $R_3$ is a heterocycle, conveniently this contains one heteroatom chosen from nitrogen, oxygen or sulphur and optionally a second nitrogen heteroatom, e.g. thienyl, furyl, pyrrolyl, pyridyl or pyrazinyl. Conveniently the heterocycle is bound to X by a ring carbon atom adjacent to a heteroatom.

$R_3$ is preferably unsubstituted phenyl.

$R_4$ and $R_5$ are conveniently hydrogen. X is conveniently —CO—.

The present invention provides a process for the production of a compound of formula I as defined above, which comprises

a) acylating a compound of formula II

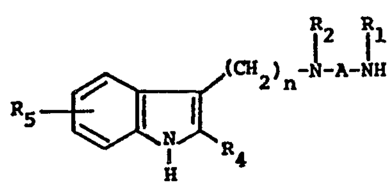

(II)

wherein n, A and $R_1$ to $R_5$ are as defined above, or

b) condensing a compound of formula III

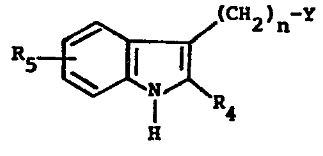

(III)

wherein n, $R_4$ and $R_5$ are as defined above, and Y is a leaving group, with a compound of formula IV

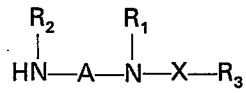

(IV)

wherein A, $R_1$ to $R_3$ and X are as defined above.

Process a) may be effected in conventional manner for the production of amides or thio-amides from amines. For example there may be used, as acylating agent, a compound of formula V

$$Z—X—R_3' \qquad (V)$$

wherein X is as defined above, $R_3'$ has the same signification as $R_3$ but is other than amino, alkylamino and optionally substituted phenylamino and Z is chlorine or bromine. The reaction may be effected conveniently in a solvent such as pyridine and at temperatures from 0°C to 25°C. Alternatively when $R_3$ is amino, alkylamino or optionally substituted phenylamino, there may be used a compound of formula VI

$$X=R_6 \qquad (VI)$$

wherein X is as defined above and $R_6$ is imino, alkylimino or optionally substituted phenylimino. The reaction may be effected conveniently in a solvent such as dimethylformamide and at temperatures from 5°C to 25°C. A compound of formula VI wherein $R_6$ is imino may be prepared in situ from potassium or sodium cyanate or thiocyanate, by treatment with acid, for example hydrochloric acid.

Process b) may be effected in conventional manner for a condensation reaction to produce a secondary or tertiary amine. Y is conveniently chlorine, bromine, iodine, tosyloxy or mesyloxy. The reaction may be conveniently effected in acetone or dimethylformamide. Suitable reaction temperatures are from 20°C to 150°C.

The compounds of formula I may be isolated from the reaction mixture and purified in known manner. The free base forms may be converted into acid addition salt forms in the usual manner and *vice versa*. Suitable acids for salt formation are hydrochloric acid, oxalic acid, fumaric acid naphthalene-2-sulphonic acid and naphthalene-1,5-disulphonic acid.

The starting material of formula II may be produced from a compound of formula III and a compound of formula VII

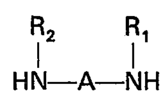

(VII)

wherein A, $R_1$ and $R_2$ are as defined above, in analogous manner to process b).

When the amine of formula VII is unsymmetrical, the conditions should be chosen to avoid the formation of the undesired corresponding compound produced by condensation at the nitrogen atom bearing the $R_1$ substituent. For this purpose the amine may be used in protected form of formula VIII

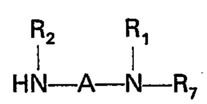

(VIII)

wherein $R_7$ is a protecting group, such as benzyl or benzyloxy, which may be removed from the resulting product, e.g. by hydrogenolysis.

A starting material of formula IIa

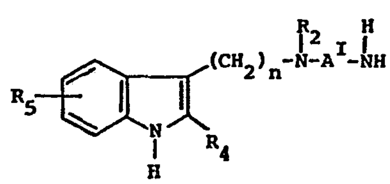

(IIa)

wherein A' is

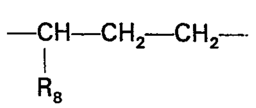

or

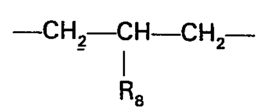

and wherein $R_8$ is $(C_{1-4})$alkyl and n, $R_2$, $R_4$ and $R_5$ are as defined above, may alternatively be produced by reducing a compound of formula IX

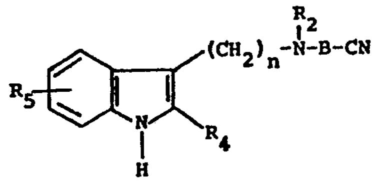

(IX)

wherein B is $—CH(R_8)—CH_2—$ or $—CH_2—CH(R_8)—$ and n, $R_2$, $R_4$, $R_5$ and $R_8$ are as defined above, e.g. by hydrogenation in the presence of Raney-nickel.

Any starting material of formula II wherein $R_1$ and/or $R_2$ is hydrogen may be converted into a corresponding compound wherein $R_1$ and $R_2$ are both alkyl, or $R_1$ is alkyl and $R_2$ is hydrogen under appropriate selective alkylation conditions.

The starting material of formula IV may be produced by acylating an amine or formula VII in analogous manner to process a). If desired, one nitrogen atom of an unsymmetrical amine may be protected to facilitate production of the desired product.

A starting material of formula IVa

$$\overset{R_2}{\overset{|}{HN}}—A''—\overset{R_1}{\overset{|}{N}}—X—R_3 \qquad \text{(IVa)}$$

wherein X, $R_2$ and $R_3$ are as defined above and A'' together with $R_1$ and the nitrogen atom to which $R_1$ is bound, form a 4-piperidyl radical, may alternatively be produced by acylating 4-piperidone with a compound of formula V or VI and condensing the resulting compound of formula X

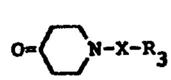

(X)

wherein X and $R_3$ are as defined above, with a compound of formula XI

$$R_2—NH_2 \qquad \text{(XI)}$$

wherein $R_2$ is as defined above, under simultaneous reduction, e.g. with hydrogen in presence of a catalyst.

Insofar as the production of any starting material is not particularly described, these are known or may be produced in conventional manner or in a manner analogous to that described above.

In the following non-limitative Examples all temperatures are indicated in degrees Centigrade.

Example 1

*N-benzoyl-N'-[3-(3-indolyl)propyl]-N'-methyl-1,3-diaminopropane*

A solution of 10.1 g benzoyl chloride in 15 ml anhydrous methylene chloride is added dropwise with stirring for 25 minutes between 0 and 10° to a solution of 15.4 g N-[3-(3-indolyl)propyl]-N-methyl-1,3-diaminopropane in 150 ml anhydrous pyridine and the reddish clear solution is stirred for 2 hours at 0°. The reaction mixture is divided between a 2N sodium carbonate solution and methylene chloride, and the organic phase is washed, dried and evaporated. Chromatographic purification of the resinous product on aluminium oxide using methylene chloride with 0.1 to 0.3% of methanol yields the title compound. The naphthalene-2-sulfonate-dihydrate, obtained by conventional methods, melts at 73—74° after crystallization from methanol/water/ethyl acetate (1:1:1).

The starting material may be obtained as follows:

a) A mixture of 57 g trifluoroacetic acid and 105 g trifluoroacetic anhydride in 400 ml anhydrous acetonitrile are added dropwise to a stirred suspension of 95.1 g 3-(3-indolyl)propionic acid in 500 ml anhydrous acetonitrile and maintained with stirring at −15° for 30 minutes. Under good cooling 500 ml anhydrous pyridine are added between −20 and −15° and quickly 238 ml of a 4.2 N solution of anhydrous methylamine in acetonitrile. The mixture is warmed with stirring at 0° for 15 minutes and maintained to 0° for 3 hours. 3-(3-indolyl)-N-methyl-propionamide (M.pt 97—98° after crystallization from methylene chloride/ethyl acetate) is obtained after working up.

b) A solution of 60.6 g 3-(3-indolyl)-N-methyl-propionamide in 500 ml anhydrous tetrahydrofuran are added dropwise at 25° for 15 minutes under nitrogen atmosphere to a suspension of 34.2 g lithium aluminium hydride in 800 ml anhydrous tetrahydrofuran and maintained at 66° for 3 hours. N-methyl-3-(3-indolyl)-propylamine (M.pt 81—82° after crystallization from methylene chloride/ethyl acetate) is obtained after working up.

c) A mixture of 37.6 g N-methyl-3-(3-indolyl)-propylamine and 21.2 g acrylonitrile in 65 ml anhydrous 1,2-dimethoxyethane are warmed with stirring at 60° for 2½ hours. N-(2-cyanoethyl)-N-methyl-3-(3-indolyl)propylamine (M.pt 48—49° after crystallization from isopropyl ether) is obtained after working up.

d) 36.2 g N-(2-cyanoethyl)-N-methyl-3-(3-indolyl)propylamine are hydrogenated at normal pressure and at room temperature with 20 g Raney-nickel catalyst in 400 ml dioxan and 400 ml of a 10% ammonia solution. N-[3-(3-indolyl)propyl]-N-methyl-1,3-diaminopropane is obtained after working up. M.pt of the neutral fumarate:180—181° (with decomposition) after crystallization from ethanol.

From the appropriate compounds of formula II the following compounds of formula I wherein X is —CO— may be obtained in analogous manner to Example 1.

| Ex. | n | $R_1$ | A | $R_2$ | $R_3$ | $R_4$ | $R_5$ | M.Pt. |
|---|---|---|---|---|---|---|---|---|
| a) | 2 | H | $-(CH_2)_3-$ | $-CH_3$ | phenyl | H | H | 122–24° 1) 10) |
| b) | 2 | H | $-(CH_2)_3-$ | $-CH_3$ | phenyl | H | $4-OC_2H_5$ | |
| c) | 3 | H | $-(CH_2)_3-$ | $-CH_3$ | phenyl | $-CH_3$ | H | |
| d) | 3 | H | $-(CH_2)_3-$ | $-CH_3$ | phenyl | H | $6-SCH_3$ | |
| e) | 3 | H | $-(CH_2)_3-$ | $-CH_3$ | phenyl | H | $5-OCH_3$ | |
| f) | 3 | H | $-(CH_2)_3-$ | $-CH_3$ | phenyl | H | $4-OCH_3$ | |
| g) | 3 | H | $-(CH_2)_3-$ | $-C_2H_5$ | phenyl | H | H | amorphous 6) |
| h) | 3 | $-CH_3$ | $-(CH_2)_3-$ | $-CH_3$ | phenyl | H | H | 124–126° 1) |
| i) | 3 | $-n-C_3H_7$ | $-(CH_2)_3-$ | $-CH_3$ | phenyl | H | H | 82–84° 1) 7) |
| j) | 3 | H | $-(CH_2)_3-$ | $-CH_3$ | benzyl | H | H | amorphous 8) |
| k) | 2 | H | 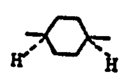 | $-CH_3$ | phenyl | H | H | 173–175° 2) 10) |
| l) | 2 | H | 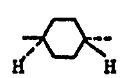 | $-CH_3$ | phenyl | H | H | 133–134° |
| m) | 3 | H | $-(CH_2)_3-$ | $-CH_3$ | 3,4,5-trimethoxy-benzyl | H | H | 77–80° 2) |
| n) | 3 | H | $-(CH_2)_3-$ | $-CH_3$ | o-chlorophenyl | H | H | amorphous 6) |

| Ex. | n | $R_1$ | A | $R_2$ | $R_3$ | $R_4$ | $R_5$ | M.Pt. |
|---|---|---|---|---|---|---|---|---|
| o) | 2 | H | $-(CH_2)_3-$ | H | diethylamino | H | H | 191–192° 3) |
| p) | 2 | H | $-(CH_2)_3-$ | $-CH_3$ | dimethylamino | H | H | 85–87° |
| q) | 2 | H | $-(CH_2)_3-$ | $-CH_3$ | p-methoxyphenyl | H | H | 133–135° 2) 10) |
| r) | 3 | H | $-(CH_2)_3-$ | $-CH_3$ | p-dimethylamino-phenyl | H | H | 107–108° |
| s) | 3 | H | $-(CH_2)_3-$ | $-CH_3$ | m-tolyl | H | H | amorphous 6) |
| t) | 2 | H | $-(CH_2)_3-$ | $-n-C_3H_7$ | p-tolyl | H | H | 181–183° 2) 10) |
| u) | 3 | H | $-(CH_2)_3-$ | $-CH_3$ | m-chlorophenyl | H | H | 133–134° 1) |
| v) | 2 | H | $-(CH_2)_3-$ | $-CH_3$ | p-chlorophenyl | H | H | 161–163° 2) 10) |
| w) | 3 | H | $-(CH_2)_3-$ | $-CH_3$ | 3,5-dimethoxyphenyl | H | H | 137–138° 2) 10) |
| x) | 3 | H | $-(CH_2)_3-$ | $-CH_3$ | 3,4,5-trimethoxy-phenyl | H | H | 103–105° 2) 10) |
| y) | 3 | H | $-(CH_2)_3-$ | $-CH_3$ | o-methoxyphenyl | H | H | amorphous 6) |
| z) | 2 | H | $-(CH_2)_3-$ | $-CH_3$ | 2-furyl | H | H | 95–96° |
| aa) | 3 | H | $-(CH_2)_3-$ | $-CH_3$ | 2-furyl | H | H | 80–82 ' 1) |
| ab) | 3 | H | $-(CH_2)_3-$ | $-CH_3$ | 2-thienyl | H | H | 82–84° 1) 7) |
| ac) | 3 | H | $-(CH_2)_3-$ | $-CH_3$ | 2-pyridyl | H | H | 122–124° 5) 10) |
| ad) | 3 | H | $-(CH_2)_3-$ | $-CH_3$ | 3-pyridyl | H | H | |
| ae) | 3 | H | $-(CH_2)_3-$ | $-CH_3$ | 4-pyridyl | H | H | 118–121° 4) 10) |
| af) | 3 | H | $-(CH_2)_3-$ | $-CH_3$ | pyrazinyl | H | H | 174–175° 9) 10) |

| Ex. | n | $R_1$ | A | $R_2$ | $R_3$ | $R_4$ | $R_5$ | M.Pt. |
|---|---|---|---|---|---|---|---|---|
| ag) | 3 | H | $-(CH_2)_3-$ | $-CH_3$ | 2-pyridylmethyl | H | H | amorphous |
| ah) | 3 | H | $-(CH_2)_3-$ | $-CH_3$ | 2-pyrrolyl | H | H | 89–91° 1) 7) |
| ai) | 2 | $-CH_3$ | $-CH_2-\underset{CH_3}{\underset{\vert}{CH}}-CH_2-$ | $-CH_3$ | phenyl | H | H | 114–115° 11) |
| aj) | 2 | H | $-(CH_2)_3-$ | $-CH_3$ | 4-hydroxyphenyl | H | H | |
| ak) | 3 | H | $-(CH_2)_3-$ | $-CH_3$ | 4-hydroxyphenyl | H | H | |
| al) | 3 | H | $-(CH_2)_3-$ | $-CH_3$ | phenyl | H | $6-CH_3$ | |
| am) | 3 | H | $-(CH_2)_3-$ | $-CH_3$ | phenyl | H | $5-CH_3$ | 148–149° 9) 10) |
| an) | 3 | H | $-(CH_2)_3-$ | $-CH_3$ | phenyl | H | $4-CH_3$ | |
| ao) | 3 | H | $-(CH_2)_3-$ | $-C_2H_5$ | phenyl | H | H | |

1) naphthalene-2-sulfonate
2) hydrogen oxalate
3) bis[base]naphthalene-1,5-disulfonate
4) dihydrobromide
5) dihydrochloride 1/2 $H_2O$
6) dihydrogen phosphate
7) monohydrate
8) bis[base]sulphate
9) bis[base]fumarate
10) with decomposition
11) hydrogen fumarate

Example 2

*N-phenylcarbamoyl-N'-[2-(3-indolyl)ethyl-N'-methyl-1,3-diaminopropane*

3 ml phenyl isocyanate are added dropwise between 5 and 10° and with stirring to a solution of 5.8 g N-[2-(3-indolyl)ethyl]-N-methyl-1,3-diaminopropane in 25 ml anhydrous dimethylformamide. The solution is stirred for an hour between 10 and 15° and evaporated. The residue is dried in high vacuum and chromatographied on silicagel using methylene chloride with 6 to 10% methanol, to yield the title compound (M.pt. of the hydrogen maleate 153—155° with decomposition after crystallization from alcohol/acetone).

The starting material may be obtained as follows:

a) Reaction of 3-[2-methylamino)ethyl]indole with acrylonitrile in dimethoxy-ethane yields the N-(2-cyanoethyl)-N-methyl-2-(3-indolyl)ethylamine which is worked up further directly.

b) Reduction of N-(2-cyanoethyl)-N-methyl-2-(3-indolyl)ethylamine with Raney-Nickel catalyst yields the N-[2-(3-indolyl)ethyl]-N-methyl-1,3-diaminopropane (M.pt. of the fumarate 153—154°).

Example 3

*N-Benzoyl-N'-[2-(3-indolyl)ethyl]-1,3-diaminopropane*

A solution of 8 g N-benzoyl-1,3-diaminopropane, 6,7 g 3-(2-bromoethyl)indole and 5 ml anhydrous triethylamine in 15 ml anhydrous dimethylformamide is maintained for 72 hours in nitrogen atmosphere. A dilute ammonia solution and methylene chloride are then added to the reaction mixture and the organic phase is dried and evaporated. The residue is chromatographied on silicagel using as eluant methylene chloride + 5% methanol + 0.3% ammonia, to yield the title compound (M.pt. of the naphthalene-2-sulfonate 203—204° with decomposition after crystallization from ethanol).

The following compounds of formula

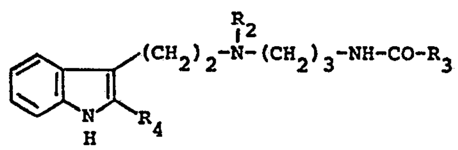

may be obtained in analogous manner to Example 3:

TABLE

| Ex. No. | $R_2$ | $R_3$ | $R_4$ | M.Pt. |
|---|---|---|---|---|
| a) | $-n-C_3H_7$ | p-tolyl | H | 181–183° 1) 3) |
| b) | $-CH_3$ | p-methoxyphenyl | H | 133–135° 1) 3) |
| c) | $-CH_3$ | p-chlorophenyl | H | 161–163° 1) 3) |
| d) | $-CH_3$ | phenyl | H | 122–124° 2) 3) |
| e) | $-CH_3$ | 2-furyl | H | 95–96° |
| f) | $-CH_3$ | phenyl | Br | 148–150° 1) 3) |

1) hydrogen oxalate

2) naphthalene-2-sulfonate

3) with decomposition

Example 4

From the appropriate 4-amino-piperidines and 2-(3-indolyl)ethyl bromide or 3-(3-indolyl)propyl bromide, the following compounds of formula

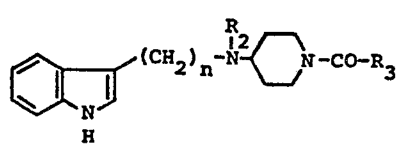

may be obtained in analogous manner to Example 3:

TABLE

| Ex. No. | n | $R_2$ | $R_3$ | M.Pt. |
|---|---|---|---|---|
| a) | 2 | $-CH_3$ | phenyl | 131–133° [3] |
| b) | 3 | $-CH_3$ | phenyl | 201–203° [1] [3] |
| c) | 2 | iso-$C_4H_9$ | phenyl | 152–154° [2] [3] |
| d) | 2 | H | phenyl | 151–152° [3] |
| e) | 2 | $-CH_3$ | phenylamino | 58–60° |
| f) | 2 | H | dimethylamino | 119–120° |

[1] naphthalene-2-sulfonate

[2] hydrochloride

[3] with decomposition

The compounds of formula I exhibit pharmacological activity in animals. In particular, the compounds exhibit anti-hypertensive activity, as indicated by standard tests, e.g. in the awake renal hypertonic Grollman rat upon administration of 1 to 50 mg/kg animal body weight of the compounds, and in the awake renal hypertonic Goldblatt dog upon administration of 1 to 10 mg/kg animal body weight of the compounds.

The compounds are therefore indicated for use as anti-hypertensives. For this use an indicated daily dose is from 10 to 2000 mg, conveniently administered in divided doses 2 to 4 times a day in unit dosage form containing from 2,5 to 1000 mg, or in sustained release form.

A particularly interesting compound is the Example 1 compound.

The compounds of formula I may be administered in pharmaceutically acceptable acid addition salt form. Such salts exhibit the same order of activity as the free base forms.

The invention also provides a pharmaceutical composition comprising a compound of formula I, in free base or pharmaceutically acceptable acid addition salt form, in association with a pharmaceutical carrier or diluent. A suitable pharmaceutical form is a capsule.

In one group of compounds n is 3, A is trimethylene, $R_1$ is hydrogen or $(C_{1-5})$alkyl, $R_2$ is hydrogen or $(C_{1-5})$alkyl, $R_3$ is phenyl or benzyl unsubstituted or mono-, di-or trisubstituted independently by halogen, hydroxy, $(C_{1-4})$alkyl, $(C_{1-4})$alkoxy or di$(C_{1-4})$alkylamino; $(C_{3-6})$cycloalkyl; or an aromatic 5- or 6-membered heterocycle containing one heteroatom chosen from nitrogen, oxygen or sulphur, $R_4$ is hydrogen, chlorine, bromine or $(C_{1-4})$alkyl, $R_5$ is hydrogen, $(C_{1-4})$alkyl, $(C_{1-4})$alkoxy, or $(C_{1-4})$alkylthio, and X is —CO—.

In another group of compounds n is 2, either A is trimethylene and $R_1$ is hydrogen or $(C_{1-5})$ alkyl, or A together with $R_1$ and the nitrogen atom to which $R_1$ is bound form a 4-piperidyl radical, and $R_2$ is hydrogen or $(C_{1-5})$alkyl, $R_3$ is $(C_{1-4})$alkyl; phenyl unsubstituted or mono-, di or trisubstituted independently by halogen, $(C_{1-4})$alkyl or $(C_{1-4})$alkoxy; $(C_{3-6})$cycloalkyl; or an aromatic 5- or 6-membered heterocycle containing one heteroatom chosen from nitrogen, oxygen or sulphur, $R_4$ is hydrogen, chlorine, bromine or $(C_{1-4})$alkyl, $R_5$ is hydrogen, $(C_{1-4})$alkyl or $(C_{1-4})$alkoxy, and X is —CO— or —CS.

## Claims

1. A compound of formula I

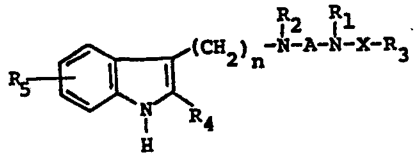

(I)

wherein n is 2 or 3,
either A is trimethylene optionally substituted by $(C_{1-4})$alkyl, or 1,4-cyclohexylidene and $R_1$ is hydrogen or $(C_{1-5})$alkyl,

or A, together with $R_1$ and the nitrogen atom to which $R_1$ is bound, form a 4-piperidyl radical,
$R_2$ is hydrogen or $(C_{1-5})$alkyl,
$R_3$ is $(C_{1-4})$alkyl; $(C_{3-6})$cycloalkyl; amino; $(C_{1-4})$alkylamino; di$(C_{1-4})$alkylamino; phenylamino wherein the phenyl ring is unsubstituted or mono-, di- or trisubstituted independently by halogen, $(C_{1-4})$alkyl, $(C_{1-4})$alkoxy or di$(C_{1-4})$alkylamino; phenyl or benzyl wherein the phenyl rings are unsubstituted or mono-, di- or trisubstituted independently by halogen, hydroxy, $(C_{1-4})$alkyl, $(C_{1-4})$alkoxy, or di-$(C_{1-4})$alkylamino; 2-, 3- or 4-pyridylmethyl; or an aromatic 5- or 6-membered heterocycle containing one heteroatom chosen from nitrogen, oxygen or sulphur and optionally an additional one or two nitrogen heteroatoms,
$R_4$ is hydrogen, chlorine, bromine or $(C_{1-4})$alkyl,
$R_5$ is hydrogen, $(C_{1-4})$alkyl, $(C_{1-4})$akoxy or $(C_{1-4})$alkylthio,
and X is —CO— or —CS—.

2. A compound according to claim 1 wherein X is —CO—.

3. A compound according to claim 1 or 2 wherein A is trimethylene optionally substituted by $(C_{1-4})$alkyl, or 2,4-cyclohexylidene.

4. A compound according to claim 1, 2 or 3 wherein A is trimethylene optionall substituted by $(C_{1-4})$alkyl.

5. A compound according to claim 3 or 4 wherein $R_1$ and $R_2$ are alkyl.

6. A compound of claim 1
wherein n is 3,
A is trimethylene,
$R_1$ is hydrogen or $(C_{1-5})$alkyl,
$R_2$ is hydrogen or $(C_{1-5})$alkyl,
$R_3$ is phenyl or benzyl unsubstituted or mono-, di- or trisubstituted independently by halogen, hydroxy, $(C_{1-4})$alkyl, $(C_{1-4})$alkoxy or di-$(C_{1-4})$alkylamino; $(C_{3-6})$cycloalkyl; or an aromatic 5- or 6-membered heterocycle containing one heteroatom chosen from nitrogen, oxygen or sulphur,
$R_4$ is hydrogen, chlorine, bromine or $(C_{1-4})$alkyl,
$R_5$ is hydrogen, $(C_{1-4})$alkyl, $(C_{1-4})$alkoxy, or $(C_{1-4})$alkylthio,
and Y is —CO—.

7. A compouind of claim 1 wherein n is 2
either A is trimethylene and $R_1$ is hydrogen or $(C_{1-5})$alkyl,
or A together with $R_1$ and the nitrogen atom to which $R_1$ is bound form a 4-piperidyl radical,
$R_2$ is hydrogen or $(C_{1-5})$alkyl,
$R_3$ is $(C_{1-4})$alkyl; phenyl unsubstituted or mono-, di- or trisubstituted independently by halogen, $(C_{1-4})$-alkyl or $(C_{1-4})$alkoxy; $(C_{3-6})$cycloalkyl; or an aromatic 5- or 6-membered heterocycle containing one heteroatom chosen from nitrogen, oxygen or sulphur,
$R_4$ is hydrogen, chlorine, bromine or $(C_{1-4})$alkyl,
$R_5$ is hydrogen, $(C_{1-4})$alkyl or $(C_{1-4})$alkoxy,
and X is —CO— or —CS—.

8. A compound according to any preceding claim wherein $R_4$ and $R_5$ are hydrogen.

9. A compound according to any preceding claim wherein $R_3$ is unsubstituted phenyl.

10. A compound of claim 1 which is N-benzoyl-N′-[3-(3-indolyl)propyl]-N′-methyl-1,3-diaminopropane.

11. A compound of claim 1 which is N-methyl-N-benzoyl-N′-methyl-N′-[2-(3-indolyl)ethyl]-2-methyl-1,3-diaminopropane.

12. A compound of any one of claims 1 to 11 in acid addition salt form.

13. A compound as claimed in any one of claims 1 to 12 for use as an anti-hypertensive.

14. A pharmaceutical composition comprising a compound according to any one of claims 1 to 11 in free base form or in pharmaceutically acceptable acid addition salt form in association with a pharmaceutical carrier or diluent.

15. A process for the production of a compound of formula I as defined in claim 1, which comprises
a) acylating a compound of formula II

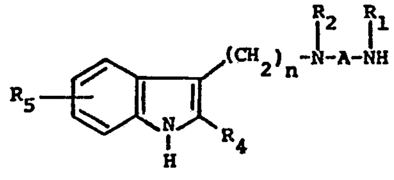

(II)

wherein n, A and $R_1$ to $R_5$ are as defined in claim 1, or
b) condensing a compound of formula III

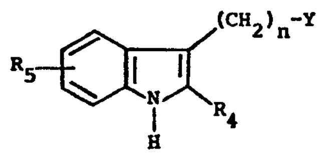

(III)

wherein n, $R_4$ and $R_5$ are as defined in claim 1, and Y is a leaving group, with a compound of formula IV

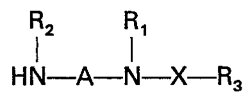

(IV)

wherein A, $R_1$ to $R_3$ and X are as defined in claim 1

**Revendications**

1. Un composé de formule I

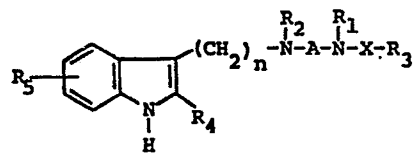

(I)

dans laquelle

n signifie 2 ou 3;

soit A signifie un groupe triméthylène éventuellement substitué par de l'alkyle contenant de 1 à 4 atomes de carbone, ou un groupe 1,4-cyclohexylidène et $R_1$ signifie l'hydrogène ou un groupe alkyle contenant de 1 à 5 atomes de carbone,

soit A, avec $R_1$ et l'atome d'azote auquel $R_1$ est rattaché, forment ensemble un radical 4-pipéridyle,

$R_2$ signifie l'hydrogène ou un groupe alkyle contenant de 1 à 5 atomes de carbone,

$R_3$ signifie un groupe alkyl contenant de 1 à 4 atomes de carbone; cycloalkyle contenant de 3 à 6 atomes de carbone; amino; alkylamino contenant de 1 à 4 atomes de carbone; dialkylamino dont les restes alkyle contiennent de 1 à 4 atomes de carbone; phénylamino dans lequel le cycle phénylique est non substitué ou mono-, di- ou trisubstitué indépendamment par des radicaux halogène, alkyl contenant de 1 à 4 atomes de carbone, alcoxy contenant de 1 à 4 atomes de carbone, alcoxy contenant de 1 à 4 atomes de carbone ou dialkylamino dont les restes alkyl continennent de 1 à 4 atomes de carbone; phényle ou benzyle dans lesquels les cycles phényliques sont non substitués ou mono-, di- ou trisubstitués indépendamment par des radicaux halogène, hydroxy, alkyl contenant de 1 à 4 atomes de carbone ou dialkylamino dont les restes alkyle contiennent de 1 à 4 atomes de carbone; 2-, 3- ou 4-pyridylméthyle; ou un hétérocycle aromatique à 5 ou 6 chaînons contenant un hétéroatome choisi parmi l'azote, l'oxygène ou le soufre et éventuellement un ou deux hétéroatomes d'azote supplémentaires,

$R_4$ signifie l'hydrogène, le chlore, le brome ou un group alkyle contenant de 1 à 4 atomes de carbone,

$R_5$ signifie l'hydrogène, un groupe alkyle contenant de 1 à 4 atomes de carbone, alcoxy contenant de 1 à 4 atomes de carbone ou alkylthio contenant de 1 à 4 atomes de carbone, et

X signifie —CO— ou —CS—.

2. Un composé selon la revendication 1, dans lequel X signifie —CO—.

3. Un compose' selon la revendication 1 ou 2, dans lequel A signifie un groupe triméthylène éventuellement substitué par de l'alkyle contenant de 1 à 4 atomes de carbone, ou un groupe 1,4-cyclohexylidène.

4. Un composé selon la revendication 1, 2 ou 3, dans lequel A signifie un groupe triméthylène éventuellement substitué par de l'alkyle contenant de 1 à 4 atomes de carbone.

5. Un composé selon la revendication 3 ou 4, dans lequel $R_1$ et $R_2$ signifient un groupe alkyle.

6. Un composé selon la revendication 1, dans lequel

n signifie 3,

A signifie un groupe triméthylène,

$R_1$ signifie l'hydrogène ou un groupe alkyle contenant de 1 à 5 atomes de carbone,

$R_2$ signifie l'hydrogène ou un groupe alkyle contenant de 1 à 5 atomes de carbone,

$R_3$ signifie un groupe phényle ou benzyle non substitués ou mono-, di ou trisubstitués indépendamment par des radicaux halogène, hydroxy, alkyle contenant de 1 à 4 atomes de carbone, alcoxy contenant de 1 à 4 atomes de carbone ou dialkylamino dont les restes alkyle contiennent de 1 à 4

atomes de carbone; cycloalkyle contenant de 3 à 6 atomes de carbone; ou un hétérocycle aromatique à 5 ou 6 chaînons contenant un hétéroatome choisi parmi l'azote, l'oxygène ou le soufre,

$R_4$ signifie l'hydrogène, le chlore, le brome ou un groupe alkyle contenant de 1 à 4 atomes de carbone,

$R_5$ signifie l'hydrogène, un groupe alkyle contenant de 1 à 4 atomes de carbone, alcoxy contenant de 1 à 4 atomes de carbone ou alkylthio contenant de 1 à 4 atomes de carbone, et

X signifie —CO—.

7. Un composé selon la revendication 1, dans lequel

n signifie 2,

soit A signifie un groupe triméthylène et $R_1$ signifie l'hydrogène ou un groupe alkyle contenant de 1 à 5 atomes de carbone,

soit A avec $R_1$ et l'atome d'azote auquel $R_1$ est rattaché, forment ensemble un radical 4-pipéridyle,

$R_2$ signifie l'hydrogène ou un groupe alkyle contenant de 1 à 5 atomes de carbone.

$R_3$ signifie un group alkyle contenant de 1 à 4 atomes de carbone; phényle non substitué ou mono-, di- ou trisubstitué indépendamment par des radicaux halogène, alkyle contenant de 1 à 4 atomes de carbone ou alcoxy contenant de 1 à 4 atomes de carbone; cycloalkyle contenant de 3 à 6 atomes de carbone; ou un hétérocycle aromatique à 5 ou 6 chaînons contenant un hétéroatome choisi parmi l'azote, l'oxygène ou le soufre,

$R_4$ signifie l'hydrogène, le chlore, le brome ou un groupe alkyle contenant de 1 à 4 atomes de carbone,

$R_5$ signifie l'hydrogène, un groupe alkyle contenant de 1 à 4 atomes de carbone ou alcoxy contenant de 1 à 4 atomes de carbone, et

X signifie —CO— ou —CS—.

8. Un composé selon l'une quelconque des revendications précédentes, dans lequel $R_4$ et $R_5$ signifient l'hydrogène.

9. Un composé selon l'une quelconque des revendications précédentes, dans lequel $R_3$ signifie un groupe phényle non substitué.

10. Un composé selon la revendication 1, qui est le N-benzoyl-N'-[3-(3-indolyl)propyl]-N'-méthyl-1,3-diaminopropane.

11. Un composé selon la revendication 1, qui est le N-méthyl-N-benzoyl-N'-méthyl-N'-[2-(3-indolyl)éthyl]-2-méthyl-1,3-diaminopropane.

12. Un composé selon l'une quelconque des revendications 1 à 11, sous forme de sel d'addition d'acides.

13. Un composé tel que revendiqué à l'une quelconque des revendications 1 à 12, pour l'utilisation comme antihypertenseur.

14. Une composition pharmaceutique comprenant un composé selon l'une quelconque des revendications 1 à 11, sous forme de base libre ou sous forme d'un sel d'addition d'acides acceptable du point de vue pharmaceutique, en association avec un véhicule ou diluant pharmaceutique.

15. Un procédé de préparation d'un composé de formule I tel que défini à la revendication 1, comprenant

a) l'acylation d'un composé de formule II

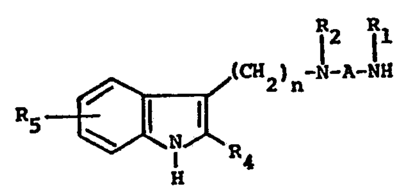

(II)

dans laquelle n, A et $R_1$ à $R_5$ sont tels que définis à la revendication 1, ou

b) la condensation d'un composé de formule III

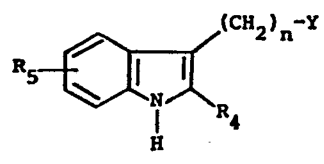

(III)

dans laquelle n, $R_4$ et $R_5$ sont tels que définis à la revendication 1, et Y signifie un groupe susceptible d'être éliminé,

avec un composé de formule IV

$$\overset{R_2}{\overset{|}{HN}}-A-\overset{R_1}{\overset{|}{N}}-X-R_3 \qquad (IV)$$

dans laquelle A, $R_1$ à $R_3$ et X sont tels que définis à la revendication 1.

**Patentansprüche**

1. Eine Verbindung der Formel I

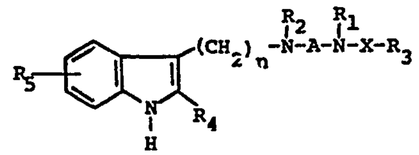

(I)

worin

n für 2 oder 3, steht

A entweder eine Trimethylengruppe, die gegebenenfalls durch Alkyl mit 1 bis 4 Kohlenstoffatomen substituiert ist, oder eine 1,4-Cyclohexylidengruppe und $R_1$ Wasserstoff oder Alkyl mit 1 bis 5 Kohlenstoffatomen bedeuten,

oder A, gemeinsam mit $R_1$ und dem Stickstoffatom, an das $R_1$ gebunden ist, einen 4-Piperidyl-Rest bildet,

$R_2$ für Wasserstoff oder Alkyl mit 1 bis 5 Kohlenstoffatomen,

$R_3$ für Alkyl mit 1 bis 4 Kohlenstoffatomen; Cycloalkyl mit 3 bis 6 Kohlenstoffatomen; Amino; Alkylamino mit 1 bis 4 Kohlenstoffatomen; Dialkylamino mit je 1 bis 4 Kohlenstoffatomen; Phenylamino wobei der Phenylring unsubstituiert oder unabhängig durch Halogen, Alkyl mit 1 bis 4 Kohlenstoffatomen, Alkoxy mit 1 bis 4 Kohlenstoffatomen oder Dialkylamino mit je 1 bis 4 Kohlenstoffatomen mono-, di- oder trisubstituiert ist; Phenyl oder Benzyl wobei der Phenylring unsubstituiert oder unabhängig voneinander durch Halogen, Hydroxy, Alkyl mit 1 bis 4 Kohlenstoffatomen, Alkoxy mit 1 bis 4 Kohlenstoffatomen oder Dialkylamino mit je 1 bis 4 Kohlenstoffatomen mono-, di- oder trisubstituiert ist; 2-, 3- oder 4-Pyridylmethyl; oder für einen aromatischen fünf- oder sechsgliedrigen Heterocyclus, der als Heteroatom Stickstoff, Sauerstoff oder Schwefel und gegebenenfalls zusätzlich 1 oder 2-Stickstoff-Heteroatom-enthält,

$R_4$ für Wasserstoff, Chlor, Brom oder Alkyl mit 1 bis 4 Kohlenstoffatomen,

$R_5$ für Wasserstoff, Alkyl mit 1 bis 4 Kohlenstoffatomen, Alkoxy mit 1 bis 4 Kohlenstoffatomen oder Alkylthio mit 1 bis 4 Kohlenstoffatomen,

und X für —CO— oder —CS— stehen.

2. Eine Verbindung gemäss Anspruch 1, worin X für —CO— steht.

3. Eine Verbindung gemäss Anspruch 1 oder 2, worin A eine Trimethylengruppe, die gegebenenfalls durch Alkyl mit 1 bis 4 Kohlenstoffatomen substituiert ist, oder eine 1,4-Cyclohexylidengruppe bedeutet.

4. Eine Verbindung gemäss Anspruch 1, 2 oder 3, worin A eine Trimethylengruppe die gegebenenfalls durch Alkyl mit 1 bis 4 Kohlenstoffatomen substituiert ist, bedeutet.

5. Eine Verbindung gemäss Anspruch 3 oder 4, worin $R_1$ und $R_2$ Alkyl bedeuten.

6. Eine Verbindung gemäss Anspruch 1, worin

n für 3,

A für Trimethylen,

$R_1$ für Wasserstoff oder Alkyl mit 1 bis 5 Kohlenstoffatomen,

$R_2$ für Wasserstoff oder Alkyl mit 1 bis 5 Kohlenstoffatomen,

$R_3$ für unsubstituiertes oder unabhängig voneinander durch Halogen, Hydroxy, Alkyl mit 1 bis 4 Kohlenstoffatomen, Alkoxy mit 1 bis 4 Kohlenstoffatomen oder Dialkylamino mit je 1 bis 4 Kohlenstoffatomen mono-, di- oder trisubstituiertes Phenyl oder Benzyl; Cycloalkyl mit 3 bis 6 Kohlenstoffatomen; oder fur einen aromatischen fünf- oder sechsgliedrigen Heterocyclus, der ein aus Stickstoff, Sauerstoff oder Schwefel ausgewähltes Heteroatom enthält,

$R_4$ für Wasserstoff, Chlor, Brom oder Alkyl mit 1 bis 4 Kohlenstoffatomen,

$R_5$ für Wasserstoff, Alkyl mit 1 bis 4 Kohlenstoffatomen, Alkoxy mit 1 bis 4 Kohlenstoffatomen, oder Alkylthio mit 1 bis 4 Kohlenstoffatomen,

und X für —CO— stehen.

7. Eine Verbindung gemäss Anspruch 1, worin

n für 2,

A entweder eine Trimethylengruppe und $R_1$ Wasserstoff oder Alkyl mit 1 bis 5 Kohlenstoffatomen bedeuten,

oder A, geminsam mit $R_1$ und dem Stickstoffatom, an das $R_1$ gebunden ist, einen 4-Piperidyl-Rest bildet,

$R_2$ für Wasserstoff oder Alkyl mit 1 bis 5 Kohlenstoffatomen,

$R_3$ für Alkyl mit 1 bis 4 Kohlenstoffatomen; unsubstituiertes oder unabhängig voneinander durch Halogen, Alkyl mit 1 bis 4 Kohlenstoffatomen oder Alkoxy mit 1 bis 4 Kohlenstoffatomen mono-, di- oder trisubstituiertes Phenyl; Cycloalkyl mit 3 bis 6 Kohlenstoffatomen; oder für einen aromatischen

fünf- oder sechsgliedrigen Heterocyclus, der ein aus Stickstoff, Sauerstoff oder Schwefel ausgewähltes Heteroatom enthält,

$R_4$ für Wasserstoff, Chlor, Brom oder Alkyl mit 1 bis 4 Kohlenstoffatomen,

$R_5$ für Wasserstoff, Alkyl mit 1 bis 4 Kohlenstoffatomen oder Alkoxy mit 1 bis 4 Kohlenstoffatomen,

und X für —CO— oder —CS— stehen.

8. Eine Verbindung gemäss einem der vorangehenden Ansprüche, worin $R_4$ und $R_5$ für Wasserstoff stehen.

9. Eine Verbindung gemäss einem der vorangehenden Ansprüche worin $R_3$ unsubstituiertes Phenyl bedeutet.

10. Eine Verbindung gemäss Anspruch 1, ist N-benzoyl-N'-[3-(3-indolyl)propyl]-N'-methyl-1,3-diaminopropan.

11. Eine Verbindung gemäss Anspruch 1, ist N-methyl-N-benzoyl-N'-methyl-N'-[2-(3-indolyl)éthyl]-2-methyl-1,3-diaminopropan.

12. Eine Verbindung gemäss einem der Ansprüche 1 bis 11, in Form ihrer Säureadditionssalze.

13. Eine Verbindung gemäss einem der Ansprüche 1 bis 12, zur Anwendung als Antihypertensivum.

14. Eine pharmazeutische Zusammensetzung, enthaltend eine Verbindung gemäss einem der Ansprüche 1 bis 11, in freier Form oder in Form ihrer pharmazeutisch verträglichen Säureadditionssalze, zusammen mit einem pharmazeutischen Träger- oder Verdünnungsmittel.

15. Ein Verfahren zur Herstellung einer Verbindung der Formel I, gemäss Anspruch 1, dadurch gekennzeichnet, dass man

a) eine Verbindung der Formel II

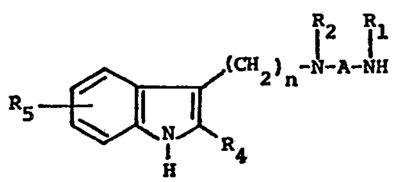

(II)

worin n, A und $R_1$ bis $R_5$ die im Anspruch 1 genannte Bedeutung besitzen, acyliert oder

b) eine Verbindung der Formel III

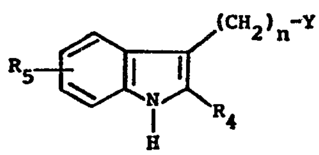

(III)

worin n, $R_4$ und $R_5$ die im Anspruch 1 genannte Bedeutung besitzen,

mit einer Verbindung der Formel IV

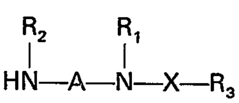

(IV)

worin A, $R_1$ bis $R_3$ und X die im Anspruch 1 genannte Bedeutung besitzen, kondensiert.